# EUROPEAN PATENT SPECIFICATION

(11) **EP 2 696 839 B1**
(45) Date of publication and mention of the grant of the patent: **26.08.2020**
(21) Application number: 12771162.0
(22) Date of filing: 02.04.2012
(51) Int. Cl.: A23D 7/00, A23D 7/005, A23D 7/06, C11B 5/00, A23L 33/115, A23L 29/25

(54) **METHODS OF MAKING OIL-IN-WATER EMULSIONS**
HERSTELLUNGSVERFAHREN FÜR ÖL-IN-WASSER-EMULSION MIT EINER MEHRFACH UNGESÄTTIGTEN FETTSÄURE
PROCÉDÉS DE FABRICATION D'ÉMULSIONS HUILE DANS L'EAU COMPRENANT UN ACIDE GRAS POLYINSATURÉ

(30) Priority: 04.04.2011 US 201113079252
(43) Date of publication of application: 19.02.2014
(73) Proprietor: DSM IP Assets B.V., 6411 TE Heerlen (NL)
(72) Inventor: WANG-NOLAN, Wei, Ellicott City, MD 21042 (US); HURST, Bradley, Martin, Odenton, MD 21113 (US); LEE, Sin, Wai, Odenton, MD 21113 (US); NEEDHAM, Micah, Winchester, KY 40391 (US); SU, Gang, Ellicott City, MD 21043 (US); MAI, Jimbin, Columbia, MD 21045 (US)
(74) Representative: Kurt, Manfred
(86) International application number: PCT/US2012/031872
(87) International publication number: WO 2012/141933

(56) References cited:
- EP-A1- 1 276 387
- WO-A1-94/01001
- WO-A1-2011/127163
- JP-A- H06 298 642
- US-A1- 2006 286 205
- US-A1- 2008 107 791
- US-A1- 2008 152 604
- US-B1- 6 261 543

## Description

### BACKGROUND OF THE INVENTION

### Field of the Invention

The present invention is directed to processes for preparing stable oil-in-water emulsions. Background

Polyunsaturated fatty acids ("PUFAs," including long-chain PUFAs "LC-PUFAs") have been shown to enhance cognitive function and maintain cardiovascular health, among other benefits. In particular, omega-3 PUFAs are important dietary components for preventing arteriosclerosis and coronary heart disease, for alleviating inflammatory conditions, and for retarding the growth of tumor cells, and omega-6 PUFAs are important as both structural lipids, and as precursors for, e.g., prostaglandins and leukotrienes. PUFAs are an important element of a healthy diet, but because PUFAs are not synthesized by humans in vivo, these compounds must be ingested. For example, the oils of many plants and animals (e.g., fish, walnuts, lingonberries, hemp, algae and the seeds and/or leaves of several plants such as flax, chia, perilla and purslane) are rich in PUFAs. Many people also choose to ingest PUFAs through dietary supplements and/or PUFA-enhanced food products. As a result, consumer demand for products that containing PUFAs has recently increased, and a wide range of products now contain PUFAs.

Oil-in-water emulsions have been used as a vehicle for PUFAs, both as a precursor for preparing comestibles, and in particular, as a component in formulated beverages. However, PUFAs present in an emulsion can become unstable and degrade (e.g., via oxidation and/or photolytic degradation), and therefore, maintaining the physical and chemical stability of PUFAs in an emulsion is critical. WO2011/127163 discloses W/O emulsions comprising tocopherol and sodium ascorbate, with a water content of 28wt% and 33wt% in the examples. US2006/286205 discloses biomass hydrolysate emulsions comprising PUFA, ascorbic acid and tocopherol. The emulsion is dried which comprises 0-30wt% water.EP1276387 discloses dried W/O emulsions comprising tuna oil encapsulated in a film forming protein. The preferred pH of the emulsions is 6,5-7,5. The powder is to be incorporated into beverages. WO94/01001 discloses dried W/O emulsions, which are micro-encapsulated oil products. Addition of tocopherol or sodium ascorbate is possible, in the examples only sodium ascorbate is applied. The powder is to be incorporated into an infant formula. JP H06 298642 discloses W/O emulsions which can comprise both water soluble and oil soluble antioxidants. Tocopherol and ascorbic acid are both listed as preferred ingredients. The emulsion is stable and is ready to use.

### BRIEF SUMMARY OF THE INVENTION

What is needed is a stable oil-in-water emulsion that is resistant to physical degradation and to oxidative degradation of a polyunsaturated fatty acid (PUFA) contained therein.

The present invention is directed to a method of making an oil-in-water emulsion comprising a) combining water and an emulsifier to provide an aqueous mixture; b) adding to the aqueous mixture a polyunsaturated fatty acid while mixing to provide an oil-in-water emulsion; and c) adding to the oil-in-water emulsion a metal chelating agent and an antioxidant; wherein the metal chelating agent is present in an amount from 3% to 20% by weight of the emulsion and wherein the antioxidant is present in an amount from about 2% to about 20% by weight of the emulsion, wherein the emulsion comprises from 2% to 6% of a salt of ascorbate or, ascorbic acid by weight of the emulsion and from 0.05% to 0.5% tocopherols by weight of the emulsion, and wherein the emulsion has a pH of 3 to 5, and wherein the water is present in a concentration of 40% to 75% by weight of the emulsion.

The present invention is also directed to an oil-in-water emulsion made by the process described above.

Further embodiments, features, and advantages of the present inventions, as well as the structure and operation of the various embodiments of the present invention, are described in detail below with reference to the accompanying drawings.

### BRIEF DESCRIPTION OF THE FIGURES

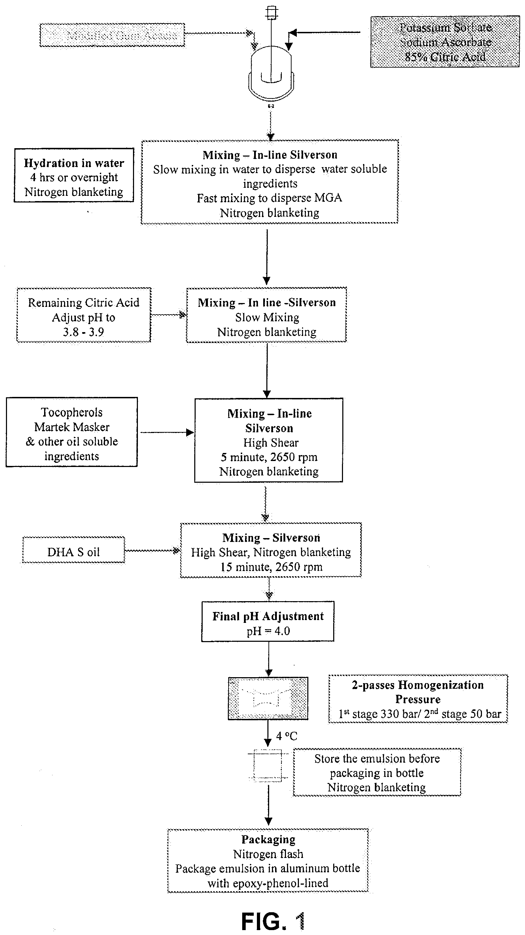

**FIG. 1** provides a processing diagram which is an example of one process by which a refrigerated emulsion can be made.

### DETAILED DESCRIPTION OF THE INVENTION

This specification discloses one or more embodiments that incorporate the features of this invention. The disclosed embodiment(s) merely exemplify the invention. The scope of the invention is not limited to the disclosed embodiment(s). The invention is defined by the claims appended hereto.

Throughout the present disclosure, all expressions of percentage, ratio, incorporation, and the like are "by weight" unless otherwise indicated. As used herein, "by weight" is synonymous with the term "by mass," and indicates that a ratio or percentage defined herein is done according to weight rather than volume, or some other measure.

As used herein, "uniformity" refers to absolute deviations from the median.

As used herein, unless otherwise stated or apparent from the context, the terms "or less" or "less than about" refers to percentages that include 0%, or amounts not detectable by current means.

As used herein, "composition" and "mixture" are used interchangeably and refer to a combination of two or more materials, substances, excipients, portions, and the like.

As used herein, "homogeneous" refers to mixtures, compositions, and, in particular, emulsions having a substantially uniform distribution, for example, of oil particles in a continuous aqueous phase. Homogeneity is synonymous with uniformity and can refer to intra-sample uniformity, batch-to-batch uniformity, and/or run-to-run uniformity. For example, intra-sample uniformity can be determined by analyzing a first portion of an emulsion, mixture, or composition and comparing this with a second portion of the same emulsion, mixture, or composition. Typical deviations of a composition (e.g., variation in the percentage by weight of excipients, the particle size, and the like) of a substantially homogeneous mixture are about 10% or less, about 5% or less, about 3% or less, about 2% or less, about 1% or less, or within experimental error.

The oil-in-water emulsions made by the process of the present invention comprise an immiscible mixture of a continuous aqueous liquid phase and a discontinuous oil phase. As used herein, "continuous aqueous liquid phase" refers to the portion of the emulsion in which the discontinuous oil phase is dispersed. Accordingly, a "discontinuous oil phase" refers to the multiplicity of discrete elements dispersed within, and immiscible with, the continuous aqueous liquid phase.

The discontinuous oil phase is present in the form of particles. As used herein, "particle" refers to an oil phase of an emulsion dispersed within a continuous liquid phase. As used herein, a "particulate" refers to an oil phase of an emulsion that comprises a plurality of discrete particles. As used herein, the term "particle size" refers to particle diameter, which is the diameter of the particles based on an approximate spherical shape of the particle based on a volumetric measurement of the particle. In addition to spherical particles, the oil-in-water emulsions of the present invention can also comprise without limitation semi-spherical, ellipsoidal and/or cylindrical particles.

As used herein, "emulsion stability" refers to the ability of an emulsion to resist changes in the physical and chemical properties of the emulsion. Examples of such changes include physical destabilization such as creaming, flocculation, coalescence, partial coalescence, phase inversion and Ostwald ripening over time and chemical changes of the emulsion formulation that lesson the ability of the emulsion to protect and stabilize a PUFA from, e.g., oxidation. Changes in physical instability are reflected in a change of one or more physical properties of the emulsion, and can include, for example, a change in the pH, viscosity, particle size and/or distribution, changes to the sedimentation properties or de-emulsification, and the like. Changes to chemical stability can include changes in organoleptic properties and the like.

For PUFA-containing emulsions, both physical and oxidative stability are important in order to achieve a shelf life under refrigerated conditions of at least 5 months, and preferably 6 months. The oil-in-water emulsions of the present invention have an extended shelf life of at least 5 months, and preferably 6 months. As used herein, "shelf-life" refers to a time period within which embodiments of emulsions can be stored and remain suitable for use, especially for consumer use. In addition to providing physical stability, the oil-in-water emulsions described herein protect and stabilize a PUFA from, e.g., oxidation.

The emulsions described herein are kinetically stable. That is, emulsion breakdown is slow, and the emulsion is able to maintain its initial state for a period of time that exceeds the intended life-time of the product. The initial state can be defined by the parameters used to measure destabilization.

Physical destabilization mechanisms include coalescence, partial coalescence, phase inversion, flocculation, Ostwald ripening, and creaming. As used herein, "coalescence" refers to a process in which two or more particles that are similar in composition come into contact with each other to form a single larger particle. As used herein, "flocculation" refers to a process in which two or more particles associate with each other, but maintain their individual integrities, and thus accelerate the rate of gravitational separation. As used herein, "Ostwald ripening" is a process in which large particles grow at the expense of smaller ones. As used herein, "creaming" is a process in which a particle which has a lower density than the surrounding liquid in an emulsion moves upward in the liquid while the particles remain separated. A creamed emulsion increases the likelihood of coalescence due to the proximity of the particles in the cream. Preferably, the physical deterioration and oxidation of an emulsion can be controlled by formulation, processing and storage conditions.

The zeta potential is one parameter by which the physical stability of an emulsion can be measured. Most emulsion droplets carry an electric charge. The continuous liquid phase surrounding the discontinuous oil phase particle exists as two parts; an inner region (Stern layer) where the ions are strongly bound to the particle and an outer (diffuse) region where the ions are less firmly associated. As used herein, "zeta potential" refers to the electric potential difference between the inner layer (Stern layer) and the outer disperse region. Zeta potential can be varied by pH, ion concentration, and water concentration. A large negative or positive Zeta potential indicates that the particles will tend to repel each other and therefore will be less likely to coalesce.

Changes in pH of the emulsion over time is a measure of emulsion stability. In a preferred embodiment, the emulsion formulation is well buffered. Such buffering enhances the ability of the emulsion formulation to withstand changes in the process or making and use, changes in its ingredients and other factors.

The oil-in-water emulsions made by the process of the present invention are particularly advantageous because the emulsions are stable over a long time. In some embodiments, the oil-in-water emulsions are substantially free from coalescence, flocculation, Ostwald ripening, and/or creaming for a period of about three months, about four months, about five months, about six months, about nine months, or about 1 year, at a temperature of about 4 °C.

As used herein, a "D₁₀" of "d(0.1)" value refers to the particle size of an oil phase, and specifically the diameter at which about 10% of all measurable particles of the oil phase have a diameter equal to or less than the D₁₀ value, and about 90% of the measurable particles have a diameter greater than the D₁₀ value.

As used herein, a "D₅₀" or "d(0.5)" value refers to the particle size of an oil phase, and specifically the diameter at which 50% of the measurable particles of the oil phase particles have a larger equivalent diameter, and the other 50% of the particles have a smaller equivalent diameter. Thus, D₅₀ generally refers to the median particle diameter.

As used herein, a "D₉₀" or "d(0.9)" value refers to the particle size of an oil phase, and specifically the diameter at which about 90% of all measurable particles of the oil phase have a diameter equal to or less than the D₉₀ value, and about 10% of the measurable particles have a diameter greater than the D₉₀ value.

As used herein, a "D₁₀₀" or "d(1.000)" value refers to the particle size of an oil phase, and specifically the diameter at which 100% of all measurable particles of the oil phase have a diameter equal to or less than the D₁₀₀ value, and 0% of the measurable particles have a diameter greater than the D₁₀₀ value.

In some embodiments, an oil-in-water emulsion described herein stored at about 4 °C for a period of 6 months or more is free from substantial variations in particle size. Preferably the oil-in-water emulsion described herein is free from substantial variations in particle size when stored at about 4 °C or colder (but not frozen). As used herein, a "substantial variation in particle size" refers to an increase in any of D₁₀, D₅₀ and/or D₉₀ of about 10% or more, for example about 20% or more, about 25% or more, about 30% or more, or about 40% or more. In some embodiments, an oil-in-water emulsion described herein can be stored for a period of 9 months or more, or 1 year or more, without a substantial variation in particle size.

Not being bound by any particular theory, it is believed that high concentrations of both a chelating agent and an antioxidant raise the zeta potential of the emulsion, thus resulting in a stable emulsion with a high zeta potential, emulsions that are substantially free from coalescence, flocculation, Ostwald ripening, and creaming, and emulsions that are free from substantial variations in particle size. In particular, the presence of sodium ascorbate is beneficial to having emulsion with a high zeta potential.

In some embodiments, an oil-in-water emulsion made by the process of the present invention further comprises a preservative. Preservatives suitable for use with the present invention include, but are not limited to, vitamin C, a tocopherol, ascorbic acid or a salt thereof (e.g., potassium sorbate). In some embodiments, the preservatives are present in an amount of less than about 7%, less than about 6%, less than about 5%, less than about 4%, less than about 3%, less than about 2%, or less than about 1%.

The oil-in-water emulsions made by the process of the present invention comprises a metal chelating agent. As used herein, "metal chelating agent" refers to compounds that bind to metal ions and form a metal/chelate complex. Metal chelating agents suitable for use with the present invention include, but are not limited to, ethylenediaminetetraacetic acid (EDTA) and salts thereof, citric acid, tartaric acid, polyphosphates (e.g., sodium hexametaphosphate, sodium acid pyrophosphate, mono sodium disodium phosphates and the like), hexametaphosphate, esters or salts, thereof, sulfite and salts thereof (e.g., sodium sulfite, potassium sulfite, and the like), bisulfite and salts thereof (e.g., sodium bisulfite and the like), cysteine hydrochloride, amino acids that have metal chelating properties, proteins that have metal chelating properties and combinations thereof. In one embodiment, the metal chelator (metal chelating agent) is sodium hexametaphosphate.

The metal chelating agent can be present in an oil-in-water emulsion of the present invention in a concentration of about 5% to 20%, about 4% to about 18%, about 5% to about 15%, about 6% to about 12%, about 7% to about 10%, or about 8% to about 9% by weight of the emulsion.

The oil-in-water emulsion made by the process of the present invention further comprises an antioxidant As used herein, the term " antioxidant refers to compounds that slow or prevent the oxidation of another chemical species, such as vitamins, pigments and lipids. Antioxidants suitable for use with the present invention include, but are not limited to, vitamin C (including fat soluble forms such as ascorbyl palmitate), vitamin E (tocopherols), a polyphenol, a phenol derivative (e.g., butylated hydroxytoluene, butylated hydroxyanisole, *tert*-butylhydroquinone, and the like), carnosic acid, lipoic acid, taurine, an aromatic carboxylic acid (e.g., cinnamic acid, benzoic acid, ascorbic acid, and the like), salts of an aromatic carboxylic acid (e.g., sodium ascorbate, potassium ascorbate, and calcium ascorbate), amino acids that have antioxidant properties, proteins that have antioxidant properties, and combinations thereof.

Suitable polyphenols for use as an antioxidant can be found in, and extracted from, a variety of foods, including plants (e.g., extracts of rosemary, cumin, grape seeds, pine bark, oats, watercress, basil, ginger, red clover, and the like), tea leaves (e.g., green tea, mate (also known as chimarrão or cimarrón), and the like), fruits (e.g., pomegranate, apple, white cherry, plum, wolfberries, blueberries, tomatoes, papaya, grapes, and the like), vegetables (e.g., alfalfa and the like), and cocoa, or can be synthesized. Exemplary polyphenols include both natural extracts and synthetic compounds. Polyphenols also include, but are not limited to, a flavone (e.g., apigenin, luteolin, tangeritin, chrysin, baicalein, scutellarein, wogonin, diosmin, flavoxate, and the like), a flavonol (e.g., 3-hydroxyflavone, azaleatin, fisetin, galangin, gossypetin, kaempferide, daempferol, isorhamnetin, morin, myricetin, natsudaidain, pachypodol, quercitin, isoquercitin, quercitrin, rhamnazin, rhamnetin, and the like), a flavanol (e.g., (+)-catechin, (-)-epicatechin, (-)-epicatchin gallate, (-)-epigallocatechin, and epigallocatechin gallate), a flavone (e.g., apigenin, luteolin, tangeritin, chrysin, baicalein, scutellarein, wogonin, diosmin, flavoxate, and the like), a flavanone, an isoflavone, a tannin, a stilbene derivative, (e.g., resveratrol and the like), an anthocyanin, an anthocyanidin, a proanthocyanidin, gallic acid, curcumin, and combinations thereof.

The antioxidant is present in an oil-in-water emulsion made by the process of the present invention in a concentration of 2% to 20%, about 3% to about 18%, about 4% to about 15%, about 5% to about 12%, about 6% to about 10%, or about 7% to about 9%, by weight of the emulsion. In some embodiments, the antioxidant is present in an amount of about 10% or less, or about 5% or less by weight of the emulsion.

The antioxidant comprises 2% to 6% of a salt of ascorbate, preferably sodium ascorbate, or, ascorbic acid, by weight of the emulsion and 0.05% to 0.5% tocopherols by weight of the emulsion. The ascorbate (ascorbic acid) helps to retard lipid oxidation by reacting with oxygen to eliminate it from food. Ascorbic acid can also help to regenerate oxidized tocopherols to the reduced state so that the tocopherols can continue to function as a free radical scavenger. Thus, the combination of ascorbic acid and tocopherol is especially advantageous and such combination results in a synergistic effect with regard to enhancing the free radical scavenger ability of the tocopherol.

In some embodiments, the ratio of metal chelating agent to antioxidant is about 10:1 to about 1:10, about 5:1 to about 1:5, about 3:1 to about 1:3, about 2:1 to about 1:2, or about 1:1.

The oil-in-water emulsions made by the process of the present invention provide safe and effective administration of a PUFA oil. As used herein, a "PUFA" ("PUFA") refers to a fatty acid having a backbone comprising 16 or more carbon atoms, (for example, 16, 18, 20 or 22 carbon atoms ("C16," "C18," "C20," or "C22," respectively)), and two or more carbon-carbon double bonds in the backbone. As used herein, a "long-chain PUFA" ("LC-PUFA") refers to a fatty acid having a backbone comprising 18 or more carbon atoms, and two or more carbon-carbon double bonds in the backbone, for example, C18:3n-3 (alpha-linolenic acid or ALA). When the notation CA:Bn-X is used for a methylene-interrupted PUFA, the "CA" is the number of carbons (for example C18, C20 or C22), B is the number of double bonds and X is the position of the first double bond counted from the methyl end of the fatty acid chain.

As used herein, the term "PUFA" encompasses the free acid form thereof, as well as salts and esters thereof. As used herein, the term "ester" refers to the replacement of the hydrogen in the carboxylic acid group of a PUFA molecule with another substituent. Typical esters are known to those in the art, a discussion of which is provided by Higuchi, T. et al., Pro-drugs as Novel Delivery Systems, Vol. 14, A.C.S. Symposium Series, Bioreversible Carriers in Drug Design, Ed. Edward B. Roche, Amer. Pharma. Assoc., Pergamon Press (1987), and Protective Groups in Organic Chemistry, McOmie ed., Plenum Press, New York (1973), each of which is incorporated herein by reference in the entirety. Examples of common esters include methyl, ethyl, trichloroethyl, propyl, butyl, pentyl, tert-butyl, benzyl, nitrobenzyl, methoxybenzyl and benzhydryl. Other esters of PUFAs are described in U.S. Patent Application Publication No. US 2010-0130608 A1, which is incorporated herein by reference in its entirety.

PUFAs for use with the present invention include omega-3, omega-6, and omega-9 fatty acids, and oxylipins derived therefrom. Exemplary omega-3 PUFAs for use with the present invention include, but are not limited to, α-linolenic acid (C18:3n-3), C18:4n-4, ω-3 eicosapentaenoic acid (20:5n-3) (eicosapentaenoic acid), ω-3 docosapentaenoic acid (docosapentaenoic acid), ω-3 docosahexaenoic acid (22:6n-3), docosatetraenoic acid (22:4n-6), and combinations thereof. Exemplary omega-6 PUFAs for use with the present invention include, but are not limited to, γ-linolenic acid, linoleic acid, conjugated linoleic acid, arachidonic acid (20:4n-6), ω-6 docosapentaenoic acid, and combinations thereof. In some embodiments, a PUFA oil for use with the present invention is all-*cis.*

In some embodiments, the PUFA comprises DHA. "DHA" refers to docosahexaenoic acid, also known by its chemical name (all-Z)-4,7,10,13,16,19-docosahexaenoic acid, as well as any salts or derivatives thereof. Thus, the term "DHA" encompasses DHA ethyl ester (DHA-EE) as well as DHA free fatty acids, phospholipids, other esters, monoglycerides, diglycerides, and triglycerides containing DHA. DHA is an ω-3 polyunsaturated fatty acid.

The term "ester" in the term "DHA-ethyl ester" refers to the replacement of the hydrogen in the carboxylic acid group of the DHA molecule with an ethyl group. In some embodiments, the ester substituent may be added to the DHA free acid molecule when the DHA is in a purified or semi-purified state. Alternatively, the DHA ester is formed upon conversion of a triglyceride to an ester.

In some embodiments, the PUFA oil that is used to make the kinetically stable emulsion is substantially free of one or more specific fatty acids. For example, a PUFA oil that contains DHA-EE can be substantially free of eicosapentaenoic acid (EPA). EPA refers to eicosapentaenoic acid, known by its chemical name (all-Z)-5,8,11,14,17-eicosapentaenoic acid, as well as any salts or derivatives thereof. Thus, the term "EPA" encompasses the free acid EPA as well as EPA alkyl esters and triglycerides containing EPA. EPA is an ω-3 polyunsaturated fatty acid. Unless otherwise stated, an oil that is used to make the kinetically stable emulsion that is "substantially free of EPA" refers to an oil in which EPA is less than about 3%, by weight, of the total fatty acid content of the oil. In some embodiments, the oil that is used to make the kinetically stable emulsion comprises less than about 2% EPA, by weight, of the total fatty acid content of the oil, less than about 1% EPA, by weight, of the total fatty acid content of the oil, less than about 0.5% EPA, by weight, of the total fatty acid content of the oil, less than about 0.2% EPA, by weight, of the total fatty acid content of the oil, or less than about 0.01% EPA by weight, of the total fatty acid content of the oil. In some embodiments, the oil has no detectable amount of EPA.

An emulsion "substantially free of EPA" refers to an emulsion in which EPA is less than about 3%, by weight, of the total fatty acid content of the emulsion. In some embodiments, the emulsion comprises less than about 2% EPA, by weight, of the total fatty acid content of the emulsion, less than about 1% EPA, by weight, of the total fatty acid content of the emulsion, less than about 0.5% EPA, by weight, of the total fatty acid content of the emulsion, less than about 0.2% EPA, by weight, of the total fatty acid content of the emulsion, or less than about 0.01% EPA by weight, of the total fatty acid content of the emulsion. In some embodiments, the emulsion has no detectable amount of EPA.

In some embodiments, the oil or emulsion containing DHA, or especially containing DHA-EE, is substantially free of docosapentaenoic acid 22:5n-6, (DPAn6). The term "DPAn6" refers to docosapentaenoic acid, omega 6, known by its chemical name (all-Z)-4,7,10,13,16-docosapentaenoic acid, as well as any salts or esters thereof. Thus, the term DPAn6 encompasses the free acid DPAn6, as well as DPAn6 ethyl esters and triglycerides containing DPAn6. DPAn6 can be removed during purification of DHA, or alternatively, the DHA can be obtained from an organism that does not produce DPAn6, or produces very little DPAn6.

As used herein, an oil "substantially free of DPAn6" refers to an oil that is used to make the emulsion that contains less than about 2%, by weight, docosapentaenoic acid 22:5n-6, (DPAn6) of the total fatty acid content of the oil. In some embodiments, the oil contains less than about 1% DPAn6, by weight, of the total fatty acid content of the oil. In some embodiments, the oil contains less than about 0.5% DPAn6, by weight, of the total fatty acid content of the oil. In some embodiments, the oil does not contain any detectable amount of DPAn6.

As used herein, an emulsion "substantially free of DPAn6" refers to an emulsion containing less than about 2%, by weight, docosapentaenoic acid 22:5n-6, (DPAn6) of the total fatty acid content of the emulsion. In some embodiments, the emulsion contains less than about 1% DPAn6, by weight, of the total fatty acid content of the emulsion. In some embodiments, the oil contains less than about 0.5% DPAn6, by weight, of the total fatty acid content of the emulsion. In some embodiments, the emulsion does not contain any detectable amount of DPAn6.

The oil or emulsion containing DHA, or especially, containing DHA-EE can also be substantially free of arachidonic acid (ARA). ARA refers to the compound (all-Z) 5,8,11,14-eicosatetraenoic acid (also referred to as (5Z,8Z,11Z,14Z)-icosa-5,8,11,14-tetraenoic acid), as well as any salts or derivatives thereof. Thus, the term "ARA" encompasses the free acid ARA as well as ARA alkyl esters and triglycerides containing ARA. ARA is an ω-6 polyunsaturated fatty acid. As used herein, an oil used to make the emulsion that is "substantially free of ARA" refers to an oil in which ARA is less than about 3%, by weight of the total fatty acid content of the oil. In some embodiments, the oil comprises, less than about 2% ARA, by weight, of the total fatty acid content of the oil, less than about 1% ARA, by weight, of the total fatty acid content of the oil, less than about 0.5% ARA, by weight, of the total fatty acid content of the oil, less than about 0.2% ARA, by weight, of the total fatty acid content of the oil, or less than about 0.01% ARA, by weight, of the total fatty acid content of the oil. In some embodiments, the oil has no detectable amount of ARA. As used herein, an emulsion "substantially free of ARA" refers to an emulsion in which ARA is less than about 3%, by weight of the total fatty acid content of the emulsion. In some embodiments, the emulsion comprises, less than about 2% ARA, by weight, of the total fatty acid content of the emulsion, less than about 1% ARA, by weight, of the total fatty acid content of the emulsion, less than about 0.5% ARA, by weight, of the total fatty acid content of the emulsion, less than about 0.2% ARA, by weight, of the total fatty acid content of the emulsion, or less than about 0.01% ARA, by weight, of the total fatty acid content of the emulsion. In some embodiments, the emulsion has no detectable amount of ARA.

A PUFA can be added to an emulsion made by the process of the present invention as a liquid (e.g., an oil), a solid (e.g., a powder), or a combination thereof.

PUFAs for use with the present invention can be isolated from any PUFA source comprising at least one PUFA capable of being dispersed in an emulsion. A PUFA for use with the present invention can be, for example, from a microbial source, a plant source, a seed source, an animal source, a fish source, or a combination thereof. PUFAs and PUFA sources suitable for use with the present invention include those described in U.S. Patent Application Publication No. 2009-0023808. For example, the PUFAs for use with the present invention can be from an oleaginous microorganism. A PUFA and/or PUFA-containing oil for use with the present invention can also be synthesized.

In some embodiments, a crude PUFA-containing oil (from, e.g., a fish, plant, seed and/or microbial source) is refined (to remove phospholipids and free fatty acids), bleached (to remove any colored bodies), enzyme-treated, and/or winterized (to remove saturated fats).

Commercially available PUFAs suitable for use with the present invention include, but are not limited to, Martek DHA™-S Oil (Martek Biosciences Corp., Columbia, Md.), Rosemary-Free Martek DHA™-S Oil (Martek Biosciences Corp., Columbia, Md.), Microalgae DHA™ Oil (Martek Biosciences Corp., Columbia, Md.), OMEGAPURE® oils (Omega Protein Corp., Houston, Tex.), MARINOL® Oils (Lipid Nutrition, Wormerveer, NL), MEG-3 oils and powders (Ocean Nutrition Corp., Dartmouth, CA), Evogel (Symrise AG, Holzminden, DE), Marine Oil (Arista Industries, Wilton, Conn.), and OMEGASOURCE® oils (Source Food Technology, Inc., Raleigh, N.C.).

In some embodiments, a PUFA oil is present in an oil-in-water emulsion made by the process of the present invention in a concentration of about 5% to about 40%, about 10% to about 30%, about 12% to about 25%, about 15% to about 20%, about 12%, about 15%, about 18%, or about 20% by weight of the emulsion.

In some embodiments, a PUFA oil is present in the discontinuous oil phase of an oil-in-water emulsion made by the process of the present invention in a concentration of about 50% to about 99%, about 60% to about 99%, about 70% to about 99%, about 80% to about 99%, about 90% to about 99%, or about 95% to about 99% by weight of the discontinuous oil phase.

In some embodiments, a PUFA is present in the oil-in-water emulsion in an amount of about 50 mg to about 80 mg per gram of emulsion, about 60 mg to about 75 mg per gram of emulsion, or about 65 mg to about 70 mg per gram of emulsion.

The continuous aqueous liquid phase of an oil-in-water emulsion made by the process of the present invention includes an aqueous liquid that is compatible with a PUFA oil and an emulsifier. Aqueous liquids suitable for use with the continuous aqueous liquid phase include, but are not limited to, water, carbonated water, syrup, diet beverages, carbonated soft drinks, fruit juices (including, but not limited to, white grape, concord dark grape, mixed berry, tropical blends, orange/pineapple/mango, strawberry/banana, pomegranate/blue berry, white grape/raspberry), vegetable juices, isotonic beverages, non-isotonic beverages, soft drinks containing fruit juice, coffee, tea, dairy products (e.g., milk, cream, and the like), soy products (e.g., milk), and the like, and combinations thereof.

Water is present in the emulsion made by the process of the present invention in a concentration of 40% to 75% by weight of the emulsion, preferably 40% to about 50%, 40% to about 45%, about 28%, about 30%, about 35%, 40%, or about 45% by weight of an emulsion.

In some embodiments, a continuous aqueous liquid phase (i.e., the aqueous liquid and any excipients soluble therein) comprises about 55% to about 95%, about 60% to about 95%, about 70% to about 90%, about 80% to about 90%, about 80% to about 90%, about 80%, about 85%, or about 90% by weight of the oil-in-water emulsion. In some embodiments, a discontinuous oil phase is present in an oil-in-water emulsion made by the process of the present invention in a concentration of about 5% to about 45%, about 5% to about 40%, about 10% to about 30%, about 10% to about 20%, about 15% to about 30%, about 15% to about 25%, about 15%, or about 20% by weight of the emulsion.

As used herein, an "emulsifier" refers to a material that promotes the stability of an oil-in-water emulsion such that the discontinuous oil phase remains substantially dispersed within the continuous aqueous liquid phase. Generally, an emulsifier is at least partially soluble in at least the continuous aqueous liquid phase or the discontinuous oil phase. In some embodiments, an emulsifier is partially soluble in both the continuous aqueous liquid phase and the discontinuous oil phase.

Emulsifiers suitable for use in the emulsions of the present invention include any emulsifier compatible with the LC-PUFAs present in the emulsions, including natural, modified, and synthetic emulsifiers, and combinations thereof. Modified emulsifiers include natural emulsifiers that are modified by chemical, enzymatic, and/or physical processes. Emulsifiers particularly suitable for use with the present invention include, but are not limited to, gum acacia, modified gum acacia (e.g., TICAMULSION®, from TIC Gums, White Marsh, Md.), a lecithin, agar, ghatti gum, modified ghatti gum, pectin, carrageenan, a xanthan gum, a modified starch, especially a modified food starch (available from, e.g., National Starch & Chemical, Bridgewater, N.J.) (for example, a modified corn starch), a modified alginate (e.g., esters of alginic acid such as propylene glycol alginate), polyoxyethylene sorbitan, a polyoxyethylene sorbitan ester (e.g., Polysorbate 20, Polysorbate 80, and the like), a sugar ester (e.g., sucrose monostearate, and the like), and combinations thereof, a fatty alcohol (e.g., cetostearyl alcohol, cetearyl alcohol, cetylstearyl alcohol, and the like), mono- and/or di-glycerides, proteins and combinations thereof. In some embodiments, the emulsifier can be a polymeric hydrocolloid, especially one that originated from a plant source. Examples of polymeric hydrocolloids that originated from a plant source include plant starches, gum arabic (gum acacia) and lignosulfonates, especially food grade lignosulfonates. In some embodiments, the emulsifier can be a modified gum acacia or a modified starch, such as an acetylated starch or starch octenyl succinate. Examples of a commercially available starch octenyl succinate include Cargill EmulTru™ 12674, which is derived from waxy maize starch. In some embodiments, the emulsifier does not have an HLB value. An "HLB" value refers to the "hydrophilic lipophilic balance" value that is an indication of the degree to which a compound is hydrophilic or lipophilic. In some embodiments, the emulsifier has an HLB value of less than 10. In some embodiments, the oil-in-water emulsion does not contain a polyglycerol fatty acid ester. In some embodiments, the emulsifier can be a natural product, such as a natural product extracted from a plant (for example, quillaja, or Q-Naturale™, sold by National Starch Food Innovation and which is derived from the quillaja tree), and combinations of emulsifiers such as those listed above.

In some embodiments, the total concentration of emulsifiers present in an oil-in-water emulsion made by the process of the present invention is about 10% to about 25%, about 10% to about 30%, about 12% to about 20%, about 14% to about 18%, about 14%, about 15%, about 16%, or about 20% or about 25% by weight of the emulsion. In some embodiments, the emulsifier is present in an amount of less than about 10% by weight of the emulsion, for example, when the emulsifier is lecithin.

Assessing whether there has been a change in the particle size distribution of the emulsion over time is a good measure of emulsion stability. A lack of change, or a small change, in particle size distribution of the emulsion over time, indicates the emulsion is stable. As used herein, a particle size "distribution" refers to the number or concentration (e.g., percentage) of particles having a certain size (i.e., diameter), or range of sizes, within a given emulsion, lot and/or batch of the present invention. Particle size and particle size distribution can be measured using Low Angle Laser Light Scattering (LALLS) with, for example, a Mastersizer Hydro 2000S (Malvern Instruments Ltd., Worcestershire, UK). Particle size and particle size distribution can also be measured by, for example, micro-photography, video microscopy, video-enhanced microscopy, Coulter counting, differential scanning calorimetry, turbidimetry, dynamic and/or static light scattering, low-intensity ultrasound, nuclear magnetic resonance, or any other particle size measurement technique known to persons of ordinary skill in the art.

In some embodiments, the discontinuous oil-phase particles have an average (median) particle size of about 20 nm to about 1.5 µm, about 50 nm to about 1 µm, about 100 nm to about 1.5 µm, about 100 nm to about 1 µm, about 150 nm to about 700 nm, or about 200 nm to about 500 nm.

In some embodiments, the discontinuous oil-phase particles have a D₉₀ of about 10 µm or less, about 5 µm or less, about 2 µm or less, or about 1 µm or less.

In some embodiments, the discontinuous oil-phase particles have a D₁₀ of about 50 nm or less, about 60 nm or less, about 70 nm or less, about 80 nm or less, about 90 nm or less, about 100 nm or less, about 200 nm or less, about 250 nm or less, about 300 nm or less, about 400 nm or less, or about 500 nm or less.

The distribution of particle sizes in a mixture can also be defined by the ratio D₁₀:D₅₀, the ratio D₁₀:D₉₀, and the ratio D₅₀:D₉₀. In some embodiments, the particle size distribution of an oil-in-water emulsion of the present invention is such that the emulsion has a ratio of D₁₀:D₅₀ of about 1:10 or less, about 1:8 or less, about 1:6 or less, about 1:5 or less, or about 1:3 or less. In some embodiments, the distribution of particle sizes in a mixture, or emulsion, can also be defined by the range of particles that are between about 0.1 µm and about 0.36 µm in diameter. In some embodiments, the percentage of particles that fall within a range of about 0.02 µm to about 0.36 µm in diameter is greater than about 95%, greater than about 96%, greater than about 97%, greater than about 98%, greater than about 99%, or 100% of the particles.

In some embodiments, it is preferred that at least 90% of the particles have a particle size between 0.02 to 0.36 microns.

In some embodiments, the viscosity of the emulsion is about 100 mPas to about 2500 mPas at 1 s⁻¹, about 100 mPas to about 2000 mPas at 1 s⁻¹, about 100 mPas to about 1400 mPas at 1 s⁻¹, about 200 mPas to about 1400 mPas at 1 s⁻¹, about 500 mPas to about 1400 mPas at 1 s⁻¹, about 700 mPas to about 1400 mPas at 1 s⁻¹, about 1000 mPas to about 1400 mPas at 1 s⁻¹, about 200 mPas to about 700 mPas at 1 s⁻¹, about 300 mPas to about 600 mPas at 1 s⁻¹, or about 400 mPas to about 500 mPas at 1 s⁻¹, Viscosity can be measured by any method known to persons of ordinary skill in the art. Unless otherwise stated, the viscosity values referred to herein were obtained at 25 °C.

In some embodiments, an oil-in-water emulsion made by the process of the present invention is acidic and in some embodiments it can be a neutral or basic pH. When potassium sulfate is used as a preservative, it is preferable that the oil-in-water emulsion be acidic. The pH of an oil-in-water emulsion can be controlled by the addition of an appropriate amount of an acid and/or a base. Acid and bases suitable for use with the present invention include, but are not limited to, acetic acid, citric acid, hydrochloric acid, sodium hydroxide, sodium carbonate, sodium bicarbonate, and the like. Not being bound by any particular theory, an acidic pH can stabilize the oil-in-water emulsion during storage. In some embodiments, anhydrous citric acid is present in an amount of 3% to 8% by weight of an emulsion.

The oil-in-water emulsion made by the process of the present invention has a pH of 3 to 5, about 3.5 to 5, or about 4 to about 4.5 or about 4.

In some embodiments, a PUFA contained in an oil-in-water emulsion made by the process of the present invention is substantially undetectable by taste and/or smell to a consumer. Thus, an emulsion made by the process of the present invention can be ingested by a consumer without any undesirable odor and/or taste. In some embodiments, an emulsion further comprises a taste-masking agent suitable for masking a scent and/or taste from an oil-in-water emulsion made by the process of the present invention or a product prepared using an oil-in-water emulsion made by the process of the present invention. An example of a taste-masking agents suitable for use with the present invention includes, but are not limited to, Martek Masker (Martek Biosciences Corp., Columbia, Md.) (supplied by Firmenich (Geneva, Switzeland) and also known as Firmenich Masker), which is a type of vanilla flavor, Givaudan (Vernier, Switzerland), International Flavors and Fragrances (New York, NY), Sensient Technologies (Milwaukee, WI), and Ogawa Flavors and Fragrances (Tokyo, Japan), and combinations thereof. In some embodiments, the taste-masking agent is present in an amount of less than 5%, less than 2%, or less than 1% by weight of an emulsion.

In addition to a PUFA, the oil-in-water emulsions made by the process of the present invention can comprise one or more excipients. As used herein, "excipient" refers to a substance useful for combining with a PUFA to provide an oil-in-water emulsion. Excipients suitable for use with the present invention meet all the requirements of the current United States and European Pharmacopeias and various other regulations and standards for pharmaceutical, food, and cosmetic additives. In generally, excipients suitable for use with the present invention are deemed safe for human consumption by the U.S. Food and Drug Administration. As used herein, "safe-for-consumption" refers to excipients, compounds, materials, and/or compositions that are, within the scope of sound judgment, suitable for contact with the tissues of human beings and animals without excessive toxicity, irritation, allergic response, or other possible complications commensurate with a reasonable benefit/risk ratio. In addition, one of skill in the art will recognize that pharmaceutically acceptable excipients can be used in the present invention including those listed in The Handbook of Pharmaceutical Excipients, 5th Ed., The Pharmaceutical Press and American Pharmacists Association, London, UK and Washington, DC (2006) .

In some embodiments, an oil-in-water emulsion made by the process of the present invention further comprises an antimicrobial agent, such as propylene glycol, potassium sorbate, or sodium benzoate. Such antimicrobial agents can be included in the composition in amounts up to the maximum allowable amount in food and/or beverage compositions. For example, compositions of the invention can include an antimicrobial agent in an amount of between about 0.05 to about 0.1% by weight of the emulsion.

In some embodiments, an oil-in-water emulsion further comprise a flavorant, which can be a synthetic, natural, fruit, or botanical flavorant, or a combination thereof. Flavorants suitable for use with the present invention include, but are not limited to, strawberry, grape, raspberry, cherry, almond, citrus fruit, orange, tangerine, lemon, lime, lemon-lime, vanilla, vanilla cream, cocoa, chocolate, coffee, kola, tea, mint, spearmint, wintergreen, menthol, licorice, butterscotch and combinations thereof.

In some embodiments, an oil-in-water emulsion further comprises a flavor enhancer, which as used herein, refers to an excipient added to achieve a better tasting product or provide a more pleasant mouth feel during administration. Non-limiting examples of flavor enhancers suitable for use with the present invention include ribotide and monosodium glutamate.

In some embodiments, an oil-in-water emulsion further comprises a natural or artificial sweetener. Suitable sweeteners include, but are not limited to, sucrose, lactose, fructose, acesulfame salts (e.g., acesulfame potassium and the like), alitame, aspartame, brazzein, curculin, cyclamic acid and salts thereof (e.g., sodium cyclamate), dihydrochalcones, glycyrrhizin and salts thereof, a mogroside, mabinlin, monatin and salts thereof, monellin, neotame, saccharin and salts thereof (e.g., saccharin sodium), siamenoside, stevia, stevioside, sucralose, thaumatin, and combinations thereof.

In some embodiments, a sweetener is present in an oil-in-water emulsion made by the process of the present invention of the present invention in a concentration of about 0.01% to about 20%, about 0.01% to about 1%, about 0.02% to about 15%, about 0.05% to about 10%, about 5% to about 20%, about 0.1% to about 5%, about 0.5% to about 4%, about 1% to about 3%, about 0.01%, about 0.05%, about 0.1%, about 1%, about 5%, or about 10% by weight of the emulsion.

In some embodiments, an oil-in-water emulsion is "sugar-free" (i.e., substantially free of a sugar and/or complex carbohydrates and/or polysaccharides that can be readily converted to a sugar in the oral cavity.

In some embodiments, an oil-in-water emulsion further comprises a colorant. A "colorant" refers to a substance that can be added to an oil-in-water emulsion to enhance and/or modify color or appearance, such as, for example, anthocyanins and oligomeric procyanidins. A colorant can also be added to an oil-in-water emulsion as a code or identifier (i.e., to indicate the concentration, intended use, and the like). Any type of colorant (i.e., "natural color" and/or "artificial color" such as F.D.&C. dyes) known to be "generally regarded as safe" (GRAS) by the FDA, and thus generally used in the confectionary trade, or otherwise approved by the FDA for use in pharmaceutical and/or nutraceutical preparations, can be used with the present invention.

In some embodiments, the discontinuous oil phase further comprises a material selected from: a terpene (e.g., limonene, pinene, and the like), a flavor oil, a vegetable oil, an essential oil, and the like, and combinations thereof. Essential oils suitable for use with the present invention include, but are not limited to, a citrus oil (e.g., an oil of lemon, orange, lime, grapefruit, mandarin, bitter orange, and the like), a leaf oil (e.g., oil or mint, peppermint, and the like), a spice oil (e.g., oil of bergamot, rosemary, and the like), a seed oil (e.g., flax seed oil, cranberry seed oil, and the like), a peel oil, and combinations thereof.

In some embodiments, an oil-in-water emulsion made by the process of the present invention further comprises a weighting agent. Weighting agents suitable for use with the present invention include, but are not limited to, a brominated oil (e.g., brominated vegetable oil), ester gum and other wood rosins, sucrose diacetate hexa-isoburtyurate (SAIB), refined gum dammar, ganuaba wax, benzyl benzoate polyglyceryl ester, glyceryl tribenzoate, and combinations thereof.

In some embodiments, a weighting agent is present in a continuous aqueous liquid phase in a concentration of about 1% to about 30%, about 2% to about 25%, or about 3% to about 20% by weight of the continuous aqueous liquid phase.

In some embodiments, an oil-in-water emulsion made by the process of the present invention of the present invention further comprises a water-dispersible or oil-dispersible bioactive. As used herein, "water dispersible bioactive" refers to materials which are both dispersible and soluble in water (or an aqueous liquid), and "oil dispersible bioactive" refers to materials which are both dispersible and soluble in an oil. Water- and/or oil-dispersible bioactives suitable for use with the present invention include, but are not limited to, an enzyme (e.g., papain), a carotenoid (e.g., β-carotene, lycopene, astaxanthin, zeaxanthin, lutein, and the like, and oxygenated variants thereof), a terpene and/or terpenoid (e.g., eucalyptol, camphor, menthol, citral, and the like), an essential oil (e.g., eugenol, gingerol, avenacoside, and the like), a phenolic acid (e.g., gallic acid, rosmarinic acid, and the like), a flavonoid (e.g., naringin, quercetin, a catechin, an anthocyanin, a coumarin, and the like), a phytoestrogen, a proanthocyanidin, a curcuminoid, a vitamin (e.g., vitamin E, Vitamin K, and the like), and combinations thereof. In some embodiments, a water dispersible bioactive is present in an oil-in-water emulsion in a concentration of about 0% to about 20%, about 0.5% to about 15%, or about 1% to about 10% by weight of the emulsion.

In some embodiments, an oil-in-water emulsion made by the method of the present invention further comprises a folded oil. Folded oils suitable for use with the present invention include, but are not limited to, 3-fold, 4-fold, 5-fold, 6-fold, 8-fold, 10-fold, 15-fold, and 20-fold oils of bergamot (including bergaptene-free bergamot oil), grapefruit (including high-aldehyde grapefruit oil and grapefruit juice extracts), lemon, lime, mandarin, orange (as well as orange juice extracts), tangerine, and the like, and combinations thereof. Folded oils suitable for use with the present invention also include washed, distilled, cold pressed, terpene-free, and/or sesquiterpene-free variants of the above exemplary folded oils.

In some embodiments, a folded oil is present in an oil-in-water emulsion made by the process of the present invention in a concentration of about 0.1% to about 10%, about 0.2% to about 5%, about 0.3% to about 1%, about 0.5% to about 5%, or about 1% to about 3% by weight of the emulsion.

In some embodiments, an oil-in-water emulsions made by the process of the present invention contains mono- and/or di-glycerides. In some embodiments, an oil-in-water emulsions of the present invention is substantially free from mono- and/or di-glycerides. An oil-in-water emulsion of the present invention that is "substantially free from mono- and/or di-glycerides" can contain, for example, about 10% or less, about 5% or less, about 1% or less, about 0.5% or less, about 0.1% or less, about 0.05% or less, or an undetectable concentration of mono- and/or di-glycerides, by weight of the emulsion.

The oil-in-water emulsions made by the process of the present invention can be used as a component or a functional ingredient in, for example, a food product, a beverage, an herbal composition, a dietary supplement, a nutritional product, a pharmaceutical composition (especially one that is administered orally or by enteral feeding), and/or a nutraceutical composition. The oil-in-water emulsions of the present invention can be present in such compositions in a concentration suitable to provide a health benefit to a consumer upon use (e.g., ingestion) of the product.

Thus, in some embodiments, the method disclosed herein comprises administering daily to the subject in need of the same, a kinetically stable emulsions of the invention, such as in a product or composition containing the same, comprising a PUFA, especially DHA, or most especially DHA-EE substantially free of eicosapentaenoic acid (EPA), wherein the DHA is derived from a non-algal source, e.g., fish.

The term "subject" refers to mammals such as humans or primates, such as apes, monkeys, orangutans, baboons, gibbons, and chimpanzees. The term "subject" can also refer to companion animals, e.g., dogs and cats; zoo animals; equids, e.g., horses; food animals, e.g., cows, pigs, and sheep; and disease model animals, e.g., rabbits, mice, and rats. The subject can be a human or non-human. The subject can be of any age. For example, in some embodiments, the subject is a human infant, i.e., post natal to about 1 year old; a human child, i.e., a human between about 1 year old and 12 years old; a pubertal human, i.e., a human between about 12 years old and 18 years old; or an adult human, i.e., a human older than about 18 years old. In some embodiments, the subject is an adult, either male or female.

As used herein, the terms "treat" and "treatment" refer to both therapeutic treatment and prophylactic or preventative measures, wherein the object is to prevent or slow down (lessen) an undesired physiological condition or disease, or obtain beneficial or desired clinical results. The term "treatment" also refers to the alleviation of symptoms associated with the above conditions or diseases.

In some embodiments, the preparation containing the PUFA provided by the emulsion of the invention is administered continuously. The term "continuous" or "consecutive," as used herein in reference to "administration," means that the frequency of administration is at least once daily. Note, however, that the frequency of administration can be greater than once daily and still be "continuous" or "consecutive," e.g., twice or even three or four times daily, as long as the dosage levels as specified herein are achieved.

In some embodiments, an oil-in-water emulsion made by the process of the invention is provided as a concentrate suitable for dilution by a local formulator, bottler, distributor, pharmacy, or other entities at the point of distribution and/or use. Concentrated oil-in-water emulsions are particularly suitable for products that must be shipped and/or stored prior to use.

As used herein, a "concentrate" refers to an oil-in-water emulsion suitable for dilution to produce a final oil-in-water emulsion having a lower concentration of emulsifier and PUFA. For example, a concentrate can comprise a beverage emulsion concentrate that can be diluted to form a beverage. In particular, the emulsion concentrate is easily dispersed within a continuous aqueous liquid phase, without further homogenization required. The formation of emulsion concentrates allows for the storage of LC-PUFAs in a stable and compact form for storage as well as transport before being dosed into a final emulsion form for consumption by a consumer. In addition, homogenization of the emulsion concentrate can be carried out in a smaller scale than homogenization of a final emulsion form to be consumed by a consumer. Thus, lower equipment costs are realized.

In some embodiments, the concentrate can be added to solids or semi-solids. For example, an emulsion concentrate may be added to solid or semi-solid foods or beverages including, but not limited to, mayonnaise, whipped topping, ice cream, yogurt, smoothies, sauces, fruit concentrate, fruit puree, baby food, and the like, specialty coffees such as frappes, etc., and teas (especially iced specialty coffees and teas that contain milk or milk products such as chai tea, Thai ice tea (cha-yen)), and the like, and combinations thereof.

In some embodiments, "carry-over" additives, such as potassium sorbate, may be present in the final product, and at a level that is considered to be "non-functional." For example, a carry through level of potassium sorbate of about 1-3 ppm when delivering 32 mg DHA in 8 oz of beverage is considered to be "non-functional." For foodstuffs, a carry-over additive is a substance, the presence of which in a given product is due solely to the fact that the additive was contained in one or more ingredients that went into the making of the product and that serve no technological function in the finished product. Processes for Preparing the Emulsions

The metal chelating agent is added after formation of the oil-in-water emulsion.

In some embodiments, an initially formed oil-in-water emulsion is homogenized by passing the oil-in-water emulsion through a homogenizer one or more times (e.g., once, twice, thrice, or 4 or more times) to form a final oil-in-water emulsion. For example, the emulsion can be passed through a homogenizer at a pressure of 10000 psi total/500 psi second stage, with 5 passes. In another example, the homogenization pressure can be 5000 psi total/750 psi second stage with 2 passes. The pressure and number of passes is determined by the homogenizer scale and type, and the final particle size that is desired.

In some embodiments a scraped surface heat exchanger (SSHE) is used with preparations or materials that have a high viscosity, especially to heat and cool the preparation and for pasteurization, if desired, for example with products that contain potassium sorbate. In some embodiments, a homogenizer is connected to the scraped surface heat exchanger.

In some embodiments the mixing is performed under nitrogen blanketing.

In some embodiments, for example, when scaling up production, it may be desirable to reduce the amount of emulsifier to avoid excessive foaming or other undesired properties. For example, an emulsion that contained 20% modified gum acacia night be modified to contain 15% modified gum acacia. Also, for production of the emulsion it may be desirable to utilize a liquid version of the emulsifier, for example, a liquid modified gum acacia, and especially a liquid modified gum acacia that has a viscosity no greater than 7000 mPas at 1 s⁻¹ at 25 °C.

Figure 1 is a process diagram that exemplifies one process for making an emulsion . As shown in Figure 1, modified gum acacia is mixed in an In-line Silverson mixer with potassium sorbate, sodium ascorbate and 85% citric acid in water to disperse the water soluble ingredients. Mixing should be at a speed sufficient to disperse the modified gum acacia. The components are allowed to hydrate in water for at least 4 hours, or overnight, under nitrogen blanketing. After hydration, and with slow mixing, under nitrogen blanketing, the citric acid is added to adjust the pH to 3.8 - 3.9. Tocopherols, Martek Masker™ and other oil soluble ingredients are added and mixed under nitrogen blanketing and high shear conditions for 5 minutes at 2650 rpm. DHA S oil is then added and the preparation mixed under nitrogen blanketing for 5 minutes at 2650 rpm. The pH is then adjusted to pH 4.0. and two passes are made to homogenize the preparation using a 1^{st} stage pressure of 330 bar and a 2^{nd} stage pressure of 50 bar. The emulsion is stored at 4 °C before packaging under nitrogen blanketing. The emulsion is then nitrogen flash packaged in an aluminum bottle with an epoxy-phenol-liner.

Having generally described the invention, a further understanding can be obtained by reference to the examples provided herein. These examples are given for purposes of illustration only and are not intended to be limiting.

### EXAMPLES

### Example 1

An oil-in-water emulsion of the present invention was prepared as follows. The amount of water was weighed and added into a mixing tank. Pre-weighed potassium sorbate, sodium ascorbate, and about 70% of the citric acid in the formulation were added while mixing. Modified gum acacia was steadily added into the mixing tank. The ingredients were then mixed in a high shear mixer at 2650 rpm until the modified gum acacia was fully dispersed and no lumps were visible. The mixer speed was then set on low (100 rpm) and mixed for at least 4 hours. The remaining amount of citric acid was then added. Pre-weighed tocoblend L70, Martek Masker, and rosemary extracts were individually added to the gum solution slowly using the in-line high shear mixer. Finally, DHA S oil was added while mixing until the oil is well mixed into the gum solution (about 2 minutes). The emulsion was then mixed for about 5 minutes at about 2650 rpm under nitrogen blanketing. The entire process was performed under nitrogen blanketing

The coarse emulsion was then homogenized at 330 bar first stage / 50 bar second stage pressure twice before packaging. The emulsion outlet temperature after homogenization was about 24 °C. Water was used to cool the emulsion at the outlet of the homogenizer. The emulsion was then bottled, purged with nitrogen and refrigerated.

**Table 1. Composition of the final emulsion prepared in Example 1.**

| **Ingredient** | **Percent (w/w)** |
|---|---|
| Martek DHA™-S Oil | 18.11% |
| Modified Gum Acacia | 18.11% |
| Potassium Sorbate | 0.07% |
| Tocoblend L70^{a} | 0.14% |
| Sodium Ascorbate | 4.75% |
| Martek Masker | 0.18% |
| Rosemary Extracts^{b} | 0.19% |
| Citric Acid | 4.63% |
| Water | to 100.00% |

| | |
|---|---|
| a: Tocoblend® L70 (Vitablend BV, Wolvega, the Netherlands) b: StabilEnhance OSR 5% from Naturex | |

The viscosity of the emulsion was between 700 and 1200 mPas at 1 rpm and at 2 rpm. The viscosity of the emulsion was measured using Brookfield DVII+ viscometer (Brookfield Engineering Inc., Middleboro, MA) using the small sample adapter with a concentric cylinder and a SC4-18 spindle. The measurement was taken at 25 °C.

The pH of the emulsion was between 3.8 and 4.2. The pH of the emulsion was measured using Orion 2 Star bench top pH meter (Thermo Fisher Scientific, Inc., Waltham, MA).

The particle size distribution was between 0.13 and 0.25 µm. Particle size distribution was measured using a Malvern Mastersizer hydro2000S (Malvern Instruments Inc., Westborough, MA).

### Example 2 (not according to the invention)

Nine emulsion formulations were made as follows.

| | Emulsion Formulation Number | | | | | | | | |
|---|---|---|---|---|---|---|---|---|---|
| Ingredients (g) | 1 | 2 | 3 | 4 | 5 | 6 | 7 | 8 | 9 |
| Martek DHA™-S oil | 50 | 50 | 50 | 50 | 50 | 50 | 50 | 50 | 50 |
| Modified gum acacia | 50 | 50 | 50 | 50 | 50 | 50 | 50 | 50 | 50 |
| Potassium sorbate | 0.1875 | 0.1875 | 0.1875 | 0.1875 | 0.1875 | 0.1875 | 0.1875 | 0.1875 | 0.1875 |
| Tocoblend™ L70 | 0.75 | 0 | 0.750 | 0.375 | 0.375 | 0 | 0.750 | 0.375 | 0 |
| Sodium ascorbate | 12.500 | 5.000 | 20.000 | 5.000 | 12.500 | 12.500 | 5.000 | 20.000 | 20.000 |
| Martek Masker | 0.5 | 0.5 | 0.5 | 0.5 | 0.5 | 0.5 | 0.5 | 0.5 | 0.5 |
| Rosemary extracts | 0.5 | 0.5 | 0.5 | 0.5 | 0.5 | 0.5 | 0.5 | 0.5 | 0.5 |
| Citric acid* | 12.8 | 8 | 18 | 8 | 12.8 | 12.8 | 8 | 18 | 18 |
| Water | 149.25 | 149.25 | 149.25 | 149.25 | 149.25 | 149.25 | 149.25 | 149.25 | 149.25 |
| Total | 276.488 | 263.438 | 289.188 | 263.813 | 276.113 | 275.738 | 264.188 | 288.813 | 288.438 |

| | Emulsion Formulation Number | | | | | | | | |
|---|---|---|---|---|---|---|---|---|---|
| Ingredients (%) | 1 | 2 | 3 | 4 | 5 | 6 | 7 | 8 | 9 |
| DHA S oil | 18.08 | 18.08 | 17.29 | 18.95 | 18.11 | 18.13 | 18.93 | 17.31 | 17.33 |
| Modified gum acacia | 18.08 | 18.08 | 17.29 | 18.95 | 18.11 | 18.13 | 18.93 | 17.31 | 17.33 |
| Potassium sorbate | 0.07 | 0.07 | 0.06 | 0.07 | 0.07 | 0.07 | 0.07 | 0.06 | 0.07 |
| Tocoblend™ L70 | 0.27 | 0 | 0.26 | 0.14 | 0.14 | 0 | 0.28 | 0.13 | 0 |
| Sodium ascorbate | 4.52 | 1.90 | 6.92 | 1.90 | 4.53 | 4.53 | 1.89 | 6.92 | 6.93 |
| Martek Masker | 0.18 | 0.19 | 0.17 | 0.19 | 0.18 | 0.18 | 0.19 | 0.17 | 0.17 |
| Rosemary extracts | 0.18 | 0.19 | 0.17 | 0.19 | 0.18 | 0.18 | 0.19 | 0.17 | 0.17 |
| Citric acid* | 4.63 | 3.04 | 6.22 | 3.03 | 4.64 | 4.64 | 3.03 | 6.23 | 6.24 |
| Water | 53.98 | 56.65 | 51.61 | 56.57 | 54.05 | 54.13 | 56.49 | 51.68 | 51.74 |

| | | | | | | | | | |
|---|---|---|---|---|---|---|---|---|---|
| *Citric acid is approximate. Each batch pH was measured and citric acid was added to pH 4.0. | | | | | | | | | |

### Example 3 Comparison of sensory evaluation

A comparison of sensory evaluations were made using Emulsion Formulation 5 (EM-5) and Emulsion Formulation 7 (EM-7), made as in Example 2 and stored refrigerated (between 4 °C to 8 °C) for 5 or 6 months as indicated below. Emulsion Formulation 5 contained about 4.64% citric acid. The % citric acid varies from 4-6% depending on the lot of modified gum acacia. The two emulsions were tested for sensory evaluation in Welch's red grape juice and Tropicana orange juice. The emulsions were added into the juices at 34 mg/serving, pasteurized at 200 °F for 15 seconds, and hot filled in HDPE bottles at 187 °F. The bottles were capped, inverted and left standing for 1 minute before cooling in ice water. The juices were stored refrigerated until sensory tasting 15 days later. The control was a canola oil emulsion that was made using Emulsion Formulation 5 but using canola oil instead of DHA S oil. The sensory test was conducted using a 7 point scale of 0 to 6, with 0 being no difference and 6 being a very large difference.

**Table 4. Sensory Evaluation of Emulsions**

| **Emulsion ID** | **Juice Type** | **Results** |
|---|---|---|
| T = 5 months | | |
| Control | Grape | - |
| EM-5 | Grape | Pass |
| EM-7 | Grape | Fail |
| Control | Orange | - |
| EM-5 | Orange | Pass |
| EM-7 | Orange | Pass |
| T=6 months | | |
| Control | Grape | - |
| EM-5 | Grape | Pass |
| EM-7 | Grape | Fail |
| Control | Orange | - |
| EM-5 | Orange | Fail |
| EM-7 | Orange | Borderline |

As shown in Table 4, formulation EM-5 containing a higher amount of sodium ascorbate and citric acid and a lower amount of Tocoblend™ L70 than formulation EM-7 possessed preferable sensory characteristics when mixed with either grape juice and orange juice at 5 months, and with grape juice at 6 months. Formulation EM-7, which contains a higher amount of Tocoblend™ L70 and a lower amount of sodium ascorbate and citric acid as compared to Formulation EM-5 possessed favorable sensory characteristics at 5 months with orange juice.

### CONCLUSION

Thus, the breadth and scope of the present invention should not be limited by any of the above described exemplary embodiments, but should be defined only in accordance with the following claims.

## Claims

1. A method of making an oil-in-water emulsion comprising
a) combining water and an emulsifier to provide an aqueous mixture;
b) adding to the aqueous mixture a polyunsaturated fatty acid while mixing to provide an oil-in-water emulsion; and
c) adding to the oil-in-water emulsion a metal chelating agent and an antioxidant;
wherein the metal chelating agent is present in an amount from 3% to 20% by weight of the emulsion and wherein the antioxidant is present in an amount from 2% to 20% by weight of the emulsion, wherein the emulsion comprises from 2% to 6% of a salt of ascorbate or ascorbic acid by weight of the emulsion and from 0.05% to 0.5% tocopherols by weight of the emulsion, and
wherein the water is present in a concentration of 40% to 75% by weight of the emulsion, and
wherein the emulsion has a pH of 3 to 5.

2. An oil-in-water emulsion made by the process of claim 1.

## Patentansprüche

1. Verfahren zur Herstellung einer Öl-in-Wasser-Emulsion, umfassend
a) Vereinigen von Wasser und einem Emulgator zur Bereitstellung einer wässrigen Mischung;
b) Zugeben einer mehrfach ungesättigten Fettsäure zu der wässrigen Mischung unter Mischen zur Bereitstellung einer Öl-in-Wasser-Emulsion und
c) Zugeben eines Metallchelatbildners und eines Antioxidans zu der Öl-in-Wasser-Emulsion;
wobei der Metallchelatbildner in einer Menge von 3 bis 20 Gew.-% der Emulsion vorliegt und wobei das Antioxidans in einer Menge von 2 bis 20 Gew.-% der Emulsion vorliegt,
wobei die Emulsion 2 bis 6 % eines Ascorbatsalzes oder Ascorbinsäure, bezogen auf das Gewicht der Emulsion, und 0,05 bis 0,5 % Tocopherole, bezogen auf das Gewicht der Emulsion, umfasst und
wobei das Wasser in einer Konzentration von 40 bis 75 %, bezogen auf das Gewicht der Emulsion, vorliegt und
wobei die Emulsion einen pH-Wert von 3 bis 5 aufweist.

2. Öl-in-Wasser-Emulsion, hergestellt nach dem Verfahren von Anspruch 1.

## Revendications

1. Procédé de préparation d'une émulsion eau-dans-huile comprenant
a) la combinaison d'eau et d'un émulsifiant pour obtenir un mélange aqueux ;
b) l'ajout au mélange aqueux d'un acide gras polyinsaturé tout en mélangeant pour obtenir une émulsion eau-dans-huile ; et
c) l'ajout à l'émulsion eau-dans-huile d'un agent chélatant métallique et d'un antioxydant ;
l'agent chélatant métallique étant présent en une quantité de 3 % à 20 % en poids de l'émulsion et l'antioxydant étant présent en une quantité de 2 % à 20 % en poids de l'émulsion, l'émulsion comprenant de 2 % à 6 % d'un sel d'ascorbate ou d'acide ascorbique en poids de l'émulsion et de 0,05 % à 0,5 % de tocophérols en poids de l'émulsion, et
l'eau étant présente en une concentration de 40 % à 75 % en poids de l'émulsion, et
l'émulsion possédant un pH de 3 à 5.

2. Émulsion eau-dans-huile préparée par le procédé selon la revendication 1.
